# EUROPEAN PATENT APPLICATION

(11) **EP 3 147 354 A1**
(43) Date of publication of application: **29.03.2017**
(21) Application number: 15796197.0
(22) Date of filing: 25.05.2015
(51) Int. Cl.: C12N 5/071

(54) **METHOD FOR PRODUCING MESOTHELIAL CELL, AND CELL SHEET FOR CONCRESCENCE PREVENTION**

(30) Priority: 23.05.2014 US 201462002448 P
(71) Applicant: The University of Tokyo, Tokyo 113-8654 (JP)
(72) Inventor: MIYAJIMA, Atsushi, Tokyo 113-8654 (JP); INAGAKI, Natsuko, Tokyo 113-8654 (JP); INAGAKI, Fuyuki, Tokyo 113-8654 (JP); KOKUDO, Norihiro, Tokyo 113-8654 (JP)
(74) Representative: Keirstead, Tanis Evelyne
(86) International application number: PCT/JP2015/064867
(87) International publication number: WO 2015/178498

(57) **Abstract**

An object of the present invention is to provide a method for differentiating mesodermal cells into mesothelium cells. The present invention provides a method for producing mesothelial cells, including a first culturing step of culturing mesodermal cells in a medium containing basic fibroblast growth factor (bFGF) and oncostatin M.

## Description

### Technical Field

The present invention relates to a method for producing mesothelial cells from mesodermal cells and a cell sheet to prevent adhesion after surgical resection, etc.

### Background Art

The number of patients suffering from primary or metastatic liver cancer is expected to increase further in future since the number of patients suffering from non-alcoholic steatohepatitis (NASH) or colon cancer has been increasing due to westernization of lifestyle.

As a treatment for liver cancer, which has a high recurrence rate, "repeated hepatectomy" has been accepted as one of the most effective treatments that can extend the survival term or from which radical cure can be expected and it has been performed widely. The repeated hepatectomy, however, has a problem of postoperative adhesion. If postoperative adhesion develops, it becomes necessary first to conduct adhesiolysis when hepatectomy is performed again. This increases the risks of prolongation of operation time, increase in blood loss, and increase in the rate of complications such as infections.

Adhesion is a phenomenon inevitably associated with abdominal surgeries and it develops irrespective of age, sex, or race. It occurs not only in the liver but also in various tissues and may cause bowel obstruction, chronic pelvic pain, abdominal pain, infertility, or the like.

Postoperative adhesion has been reported to develop at a high frequency of 93 to 100% after surgical operations, including mild cases, and various measures and prevention have so far been proposed. Such measures include, for example, adhesiolysis by reoperation by laparotomy or laparoscopic surgery, of which the former puts an excessive burden on the patient and the latter requests high skills to the surgeon. Moreover, it is a problem that new adhesion may develop due to the reoperation itself.

Efforts to alleviate postoperative adhesion by medication have been made, but little effects have been observed so far.

Now, the most common measures against postoperative adhesion include application of an absorbable adhesion preventing material made of a polymer material. Application of an absorbable adhesion preventing material is reported to alleviate adhesion (Non Patent Literature 1). However, recent meta-analyses have revealed that the frequency of bowel obstruction itself does not decrease and that the frequency of the intraperitoneal abscess and the failure of suture increases (Non Patent Literature 2 and 3). Furthermore, it has been pointed out that absorbable adhesion preventing agents may inhibit wound healing and increase the risk of postoperative bile leakage when used for liver.

Moreover, repeated hepatectomy has a problem of decreased hepatic functional reserve of remnant liver besides the problem of adhesion. It is a problem that decrease in reserved liver function leads to liver failure and therefore limits the number of times and the extent of resection of repeated hepatectomy.

In order to solve such problems, the present inventors have developed cell sheets made from mesothelial cells to place on the resected surface after hepatectomy (Patent Literature 1).

### Citation List

### Patent Literature

Patent Literature 1: International Publication No. WO 2014/115776

### Non Patent Literature

Non Patent Literature 1: Fazio, VW. et al., Dis Colon Rectum, 49, 1-11, 2006
Non Patent Literature 2: Tang, CL. et al., Ann Surg., 243, 449-455, 2006
Non Patent Literature 3: Zeng, Q. et al., World J Surg. , 31, 2125-2131, 2007

### Summary of Invention

### Technical Problem

The mesothelial cell sheet developed by the present inventors is very useful, and supply becomes easier if mesothelial cells can be induced from stem cells such as iPS cells. Therefore, it is an object of the present invention to provide a method for differentiating mesodermal cells into mesothelial cells, etc.

### Solution for Problems

The present inventors have studied to solve the aforementioned problems and found that differentiation into mesothelial cells can be induced by culturing mesodermal cells in a medium supplemented with bFGF and oncostatin M. Moreover, it has been confirmed that sorting cells by using a molecule such as PCLP1, mesothelin, or ALCAM as a marker during culturing increases directed differentiation into mesothelial cells and allow efficient production of mesothelial cells.

Furthermore, the present inventors confirmed that mesothelial cells can be obtained by a similar method even using mesodermal cells derived from pluripotent stem cells and cell sheets made from the obtained mesothelial cells are useful for preventing postoperative adhesion on the resected surface after hepatectomy, thereby completing the present invention.

Accordingly, the present invention relates to:
[1] A method for producing mesothelial cells, comprising a first culturing step of culturing mesodermal cells in a medium containing basic fibroblast growth factor (bFGF) and oncostatin M (OSM);
[2] The method for producing mesothelial cells according to the aforementioned [1], further comprising, after the first culturing step, a first sorting step selected from the following (i) and (ii):
   (i) a step of sorting podocalyxin-like protein 1 (PCLP1)-positive and mesothelin-positive cells; and
   (ii) a step of sorting PCLP1-positive and activated-leukocyte cell adhesion molecule (ALCAM)-positive cells;
[3] The method for producing mesothelial cells according to the aforementioned [2], further comprising, after the first sorting step, a second culturing step of culturing the cells in a medium containing bFGF and oncostatin M;
[4] The method for producing mesothelial cells according to the aforementioned [3], wherein the medium of the second culturing step further comprises insulin-transferrin-selenium-X (ITS-X);
[5] The method for producing mesothelial cells according to the aforementioned [3] or [4], wherein the first sorting step is that of (i) and the method further comprises, after the second culturing step, a second sorting step of sorting PCLP1-positive and ALCAM-positive cells;
[6] The method for producing mesothelial cells according to any one of the aforementioned [1] to [5], wherein the mesothelial cells are liver mesothelial cells;
[7] The method for producing mesothelial cells according to any one of the aforementioned [1] to [6], wherein the mesodermal cells are cells differentiated in vitro from pluripotent stem cells;
[8] The method for producing mesothelial cells according to the aforementioned [7], wherein the in vitro differentiation from the pluripotent stem cells to the mesodermal cells is achieved by suspension culture;
[9] The method for producing mesothelial cells according to the aforementioned [7] or [8], wherein the in vitro differentiation from the pluripotent stem cells to the mesodermal cells is performed such that embryoid bodies are formed;
[10] A cell population of mesothelial cells produced by a method according to any one of the aforementioned [1] to [9], wherein the cell population comprises 10% or more of PCLP1-positive and ALCAM-positive cells;
[11] A method for producing a cell sheet for preventing adhesion, comprising the steps of culturing the cell population according to the aforementioned [10] in a medium containing bFGF and oncostatin M; and producing a cell sheet with the cell population;
[12] The method for producing a cell sheet for preventing adhesion according to the aforementioned [11], wherein the medium further comprises ITS-X;
[13] A method for producing a cell sheet for preventing adhesion, comprising the steps of: selecting PCLP1-positive cells from cultured cells; and producing a cell sheet from the selected cells;
[14] A method for producing a cell sheet for preventing adhesion, comprising the steps of: selecting PCLP1-positive and ALCAM-positive cells from cultured cells; and producing a cell sheet from the selected cells;
[15] A cell sheet for preventing adhesion produced by a method according to any one of the aforementioned [11] to [14]; and
[16] A cell sheet for preventing adhesion, consisting of a cell sheet comprising 50% or more of PCLP1-positive and ALCAM-positive cells.

### Advantageous Effects of Invention

According to a method according to the present invention, mesothelial cells can be induced from mesodermal cells simply by adding bFGF and oncostatin M to their medium. Mesodermal cells differentiated from pluripotent stem cells can be also used in this method.

Mesothelial cells produced by a method according to the present invention are useful as an adhesion preventing material on the resected surface after hepatectomy, etc.

### Brief Description of Drawings

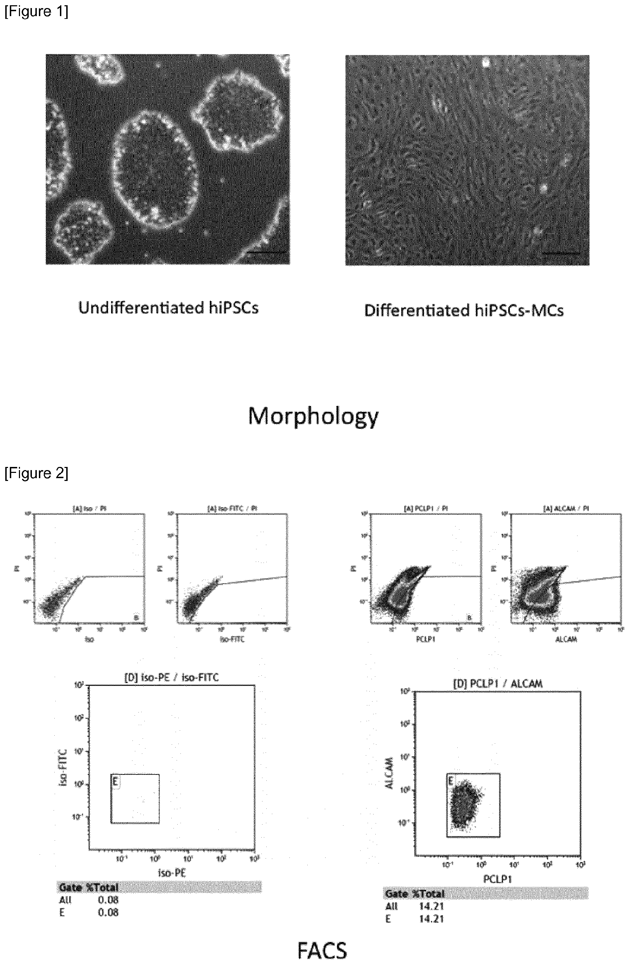

[Figure 1] Figure 1 illustrates a phase contrast microscope photograph (left panel) of human iPS cells in an undifferentiated state and a phase contrast microscope photograph (right panel) of liver mesothelial precursor cells obtained by culturing mesodermal cells induced from human iPS cells in the presence of oncostatin M and bFGF, sorting ALCAM-positive and PCLP1-positive cells, and further culturing the cells. The scale bar is 100 µm.
[Figure 2] Figure 2 illustrates results of the sorting with a cell sorter using ALCAM and PLCP1 as markers described for Figure 1.

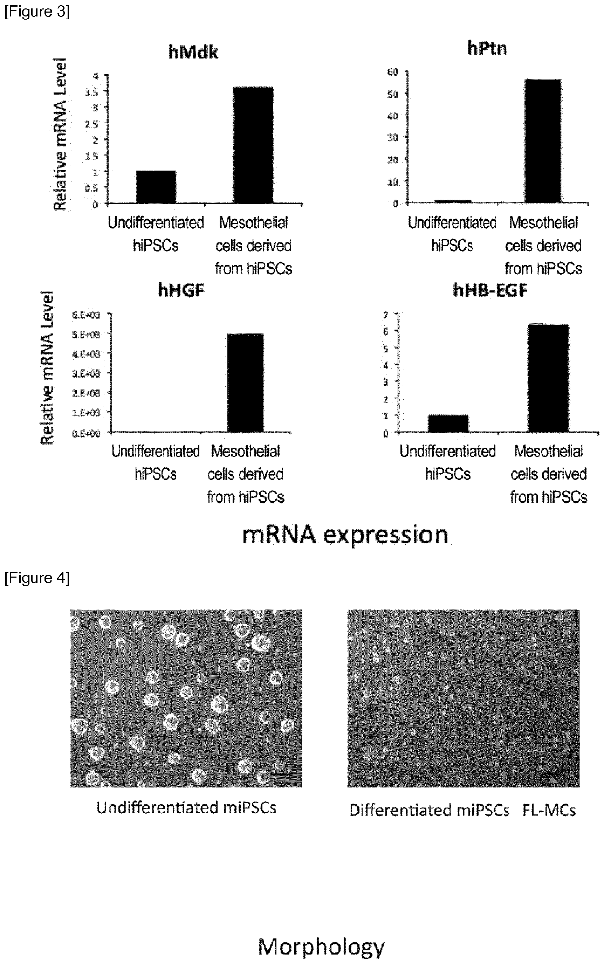

[Figure 3] Figure 3 illustrates results of gene expression analysis of the liver mesothelial precursor cells derived from human iPS cells illustrated in the right panel in Figure 1.
[Figure 4] Figure 4 illustrates the morphology of liver mesothelial precursor cells derived from murine iPS cells. Differentiation from murine iPS cells into liver mesothelial precursor cells was induced. Phase contrast microscope photographs of undifferentiated murine iPS cells and cells obtained by sorting an ALCAM-positive and PCLP1-positive fraction from induced cells and culturing the fraction. The scale bar is 100 µm.

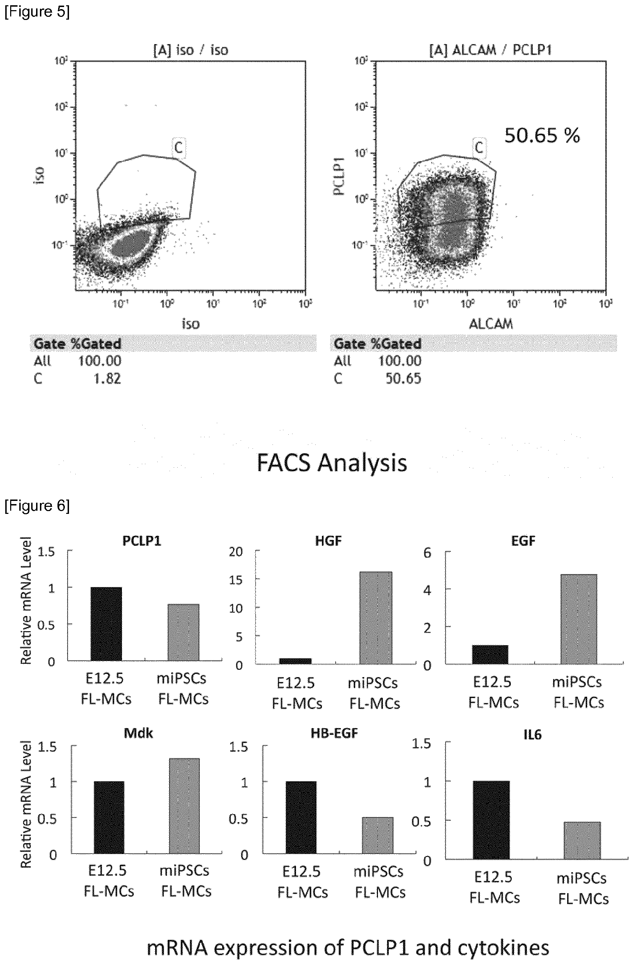

[Figure 5] Figure 5 illustrates results of FACS analysis of cells on Day 23 of the induction of differentiation from murine iPS cells using anti-murine ALCAM and anti-PCLP1 antibodies.
[Figure 6] Figure 6 illustrates results of gene expression analysis of liver mesothelial precursor cells derived from murine iPS cells. The gene expression of liver mesothelial precursor cells derived from murine iPS cells on Day 28 of the induction and the gene expression of embryonic liver mesothelial cells isolated at E12.5 and cultured were compared.

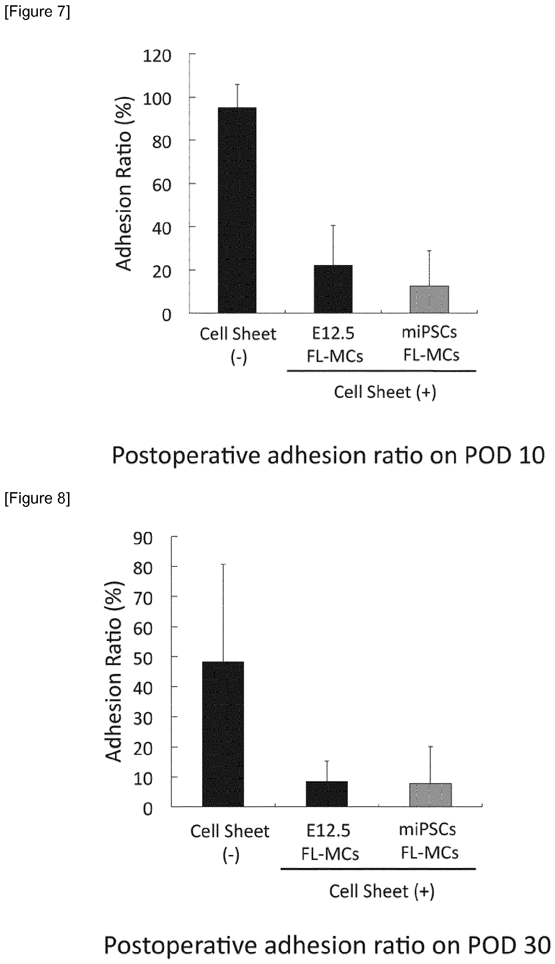

[Figure 7] Figure 7 illustrates results of examination on the adhesion preventing effect on Day 10 after applying a sheet of liver mesothelial precursor cells derived from murine iPS cells. Sheets of liver mesothelial cells derived from murine iPS cells were produced from liver mesothelial cells derived from murine iPS cells on temperature-responsive culture dishes and applied to liver resected surfaces of a mouse model of posthepatectomy adhesion established earlier. On Day 10, laparotomy was performed and the adhesion ratio was determined. The adhesion ratio was compared with those of the sheet non-application group and the E12. 5 embryonic liver mesothelial cell sheet-application group.
[Figure 8] Figure 8 illustrates results of examination on the adhesion preventing effect on Day 30 after applying a sheet of liver mesothelial precursor cells derived from murine iPS cells. Sheets of liver mesothelial cells derived from murine iPS cells were applied to liver resected surfaces of a mouse model of posthepatectomy adhesion. On Day 30, laparotomy was performed and the adhesion ratio was determined.

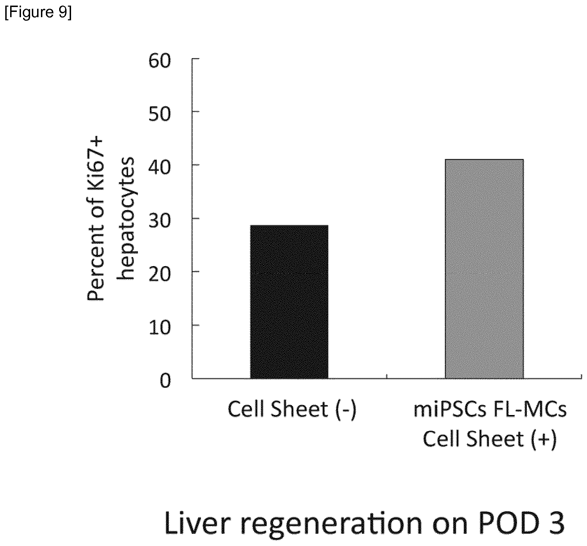

[Figure 9] Figure 9 illustrates results of examination on the liver regeneration promoting effect of sheets of liver mesothelial precursor cells derived from murine iPS cells. Sheets of liver mesothelial cells derived from murine iPS cells were applied to liver resected surfaces of a mouse model of posthepatectomy adhesion. On Day 3, laparotomy was performed and the percent of Ki67-positive hepatocytes was determined by imaging cytometry.

### Description of Embodiments

### (Method for producing mesothelial cells)

A method for producing mesothelial cells according to the present invention includes a first culturing step of culturing mesodermal cells in a medium containing basic fibroblast growth factor (bFGF) and oncostatin M for differentiation. Cells expressing a mesothelial cell marker can be obtained by this step.

The term "mesothelial cells," as used herein, refers to monolayer cells that cover the uppermost surface of the mesothelium (patterned tissue covering surfaces of body cavities such as the thorax, the pericardium, and the abdominal cavity). As used herein, the term "mesothelial cells" includes mesothelial precursor cells.

Origins of the mesodermal cells are not particularly limited and the mesodermal cells may be those extracted from the living body or those differentiated from pluripotent stem cells. The pluripotent stem cells, as used herein, are stem cells having the pluripotency, with which cells can differentiate into any cells present in the living body, and the proliferative capacity and examples include, but are not limited to, embryonic stem cells (ES cells), embryonic stem cells derived from an embryo cloned by nuclear transfer or nuclear transfer embryonic stem cells (ntES cells), germline stem cells (GS cells), embryonic germ cells (EG cells), induced pluripotent stem cells (iPS cells; for example, Takahashi, K. and Yamanaka, S., Cell, 126 663-676, 2006), and pluripotent cells derived from cultured fibroblasts or bone marrow stem cells or multi-lineage differentiating stress enduring cells (Muse cells).

Differentiation from pluripotent stem cells into mesodermal cells can be performed according to a known method, for example, a method involving conducting suspension culture and forming embryoid bodies (EB). Mesodermal cells are characterized by mesodermal cell markers such as platelet-derived growth factor receptor α (PDGFRα) and vascular endothelial growth factor receptor 2 (VEGFR2).

The medium of the first culturing step contains bFGF and oncostatin M. As long as the mesodermal cells differentiate into mesothelial cells, any required substance other than bFGF and oncostatin M may be added as appropriate. The concentrations of the substances added to the medium can be determined by a person skilled in the art as appropriate. For example, the concentration of the bFGF can be 1 ng/mL to 100 ng/mL, 2 ng/mL to 50 ng/mL, 3 ng/mL to 25 ng/mL, or about 10 ng/mL. The concentration of oncostatin M can be 1 ng/mL to 100 ng/mL, 2 ng/mL to 50 ng/mL, 3 ng/mL to 25 ng/mL, or about 10 ng/mL. Culture conditions such as culture temperature and culture period can be also selected as appropriate.

The method for producing mesothelial cells according to the present invention may include, after the first culturing step, a first sorting step of sorting mesothelial cell marker-positive cells with a cell sorter. The mesothelial cell marker is not particularly limited, but examples include podocalyxin-like protein 1 (PCLP1), mesothelin, and activated-leukocyte cell adhesion molecule (ALCAM). According to one embodiment, the first sorting step may include sorting PCLP1-positive and mesothelin-positive cells. This enables acquisition of a cell population that undergoes highly directed differentiation into mesothelial cells. When PCLP-positive and mesothelin-positive cells are selected in the first sorting step, a second sorting step described later may be further conducted. This embodiment may be employed when murine cells are used. According to another embodiment, the first sorting step may include sorting PCLP1-positive and ALCAM-positive cells. This embodiment may be used when human cells are used.

The present invention encompasses cell populations obtained by sorting in the first sorting step. Such a cell population is inducted to differentiate from mesodermal cells and contains, in one embodiment, 10% or more, 20% or more, 30% or more, 40% or more, 50% or more, 60% or more, 70% or more, 80% or more, or 90% or more of PCLP1-positive and mesothelin-positive cells. In another embodiment, the population contains 10% or more, 20% or more, 30% or more, 40% or more or 50% or more, 60% or more, 70% or more, 80% or more, or 90% or more of PCLP1-positive and ALCAM-positive cells. The percentage that PCLP1-positive and mesothelin-positive cells or PCLP1-positive and ALCAM-positive cells and the like comprise in a certain cell population can be determined by a person skilled in the art as appropriate, for example, using a cell sorter.

The method for producing mesothelial cells according to the present invention may further include, after the first sorting step, a second culturing step of culturing cells in a medium containing bFGF and oncostatin M. Any required substance other than bFGF and oncostatin M may be also added to this medium as appropriate unless the substance interferes with differentiation of mesodermal cells into mesothelial cells. For example, insulin-transferrin-selenium-X (ITS-X) may be further added to the medium of the second culturing step.

The method for producing mesothelial cells according to the present invention may include, after the second culturing step, a second sorting step of sorting cells with a mesothelial cell marker to obtain a mesothelial cell population having a higher purity. Using a mesothelial cell marker at the embryonic stage as a mesothelial cell marker in this step enables acquisition of a cell population exhibiting a tendency similar to immature mesothelial cells obtained from an embryo. Known mesothelial cell markers include, for example, podocalyxin-like protein 1 (PCLP1; Onitsuka I., Tanaka M. , and Miyajima A. Gastroenterology 138, 1525-1536, 2010). PCLP1 is also referred to as podocalyxin-like (PODXL) or podocalyxin-like protein (PCLP), etc. Cell sheets for preventing adhesion made from this cell population can promote not only prevention of adhesion but also regeneration of the tissue or organ. According to one embodiment, the first sorting step may involve sorting PCLP1-positive and mesothelin-positive cells and the second sorting step may involve sorting PCLP1-positive and ALCAM-positive cells.

The present invention encompasses cell populations obtained by sorting in the second sorting step. Such a cell population is induced to differentiate from mesodermal cells and contains 10% or more, 20% or more, 30% or more, 40% or more, 50% or more, 60% or more, 70% or more, 80% or more, or 90% or more of PCLP1-positive and ALCAM-positive cells.

The method for producing mesothelial cells according to the present invention can be applied to any mesothelial cell. For example, the method can be a method for producing liver mesothelial cells or liver mesothelial precursor cells. As used herein, the term "liver mesothelial precursor cells" refers to cells corresponding to embryonic liver mesothelial cells and to liver mesothelial cells separated from embryonic liver on around Day 11 to 13 of pregnancy or cells differentiated in vitro to the same stage as liver mesothelial cells separated from embryonic liver on Day 11 to 13 of pregnancy.

### (Cell sheet for preventing adhesion and method for producing the same)

The present invention also encompasses cell sheets for preventing adhesion and methods for producing the same. The cell sheets for preventing adhesion according to the present invention are useful as adhesion preventing materials for suppressing adhesion in an organ or tissue after surgical operation, etc. The cell sheets for preventing adhesion may contain supports necessary for maintaining the shape of their cell cultures or cell sheets. In another embodiment, a cell sheet for preventing adhesion may substantially consist of a cell sheet. The cell sheet substantially consisting of a cell sheet can reproduce a state more similar to the natural state in the living body and therefore is advantageous in that a function of promoting regeneration of an organ and tissue is more easily obtained.

In one embodiment, the method for producing a cell sheet for preventing adhesion according to the present invention can produce the cell sheet from mesothelial cells produced by a method for producing mesothelial cells according to the present invention by a known method for producing a cultured cell sheet or a method similar thereto.

The "mesothelial cells produced by a method for producing mesothelial cells according to the present invention" may be any of cells obtained as a result of the first culturing step, cells obtained as a result of the first sorting step, cells obtained as a result of the second culturing step, cells obtained as a result of the second sorting step, or cells obtained as a result of subsequent further culturing.

According to one embodiment, the "mesothelial cells produced by a method for producing mesothelial cells according to the present invention" may be cells obtained by conducting the first culturing step, sorting PCLP1-positive and ALCAM-positive cells as the first sorting step, and conducting the second culturing step (in the presence or absence of ITS-X).

According to another embodiment, the "mesothelial cells produced by a method for producing mesothelial cells according to the present invention" may be cells obtained by conducting the first culturing step, sorting PCLP1-positive and mesothelin-positive cells as the first sorting step, conducting the second culturing step (in the presence or absence of ITS-X), sorting of PCLP1-positive and ALCAM-positive cells as the second sorting step, and conducting or not conducting a subsequent third culturing.

In one embodiment, the method for producing a cell sheet for preventing adhesion according to the present invention include the steps of selecting PCLP1-positive cells from cultured cells and producing a cell sheet from the selected cells. In this method, PCLP1-positive and ALCAM-positive cells may be selected. As illustrated in Examples described later, cell sheets produced by selecting such cells conveniently function as adhesion preventing materials.

According to an example of the method for producing a cell sheet for preventing adhesion, a cell sheet may be obtained by culturing (i) mesothelial cells obtained by a method for producing mesothelial cells according to the present invention, (ii) PCLP1-positive cells, or (iii) PCLP1-positive and ALCAM-positive cells on a culture dish coated with a temperature-responsive polymer; and after mesothelial cells become a sheet, collecting the sheet from the culture dish by changing temperature. Also in the culturing step with the culture dish coated with a temperature-responsive polymer, bFGF and oncostatin M may be added and ITS-X may be further added to the medium.

Examples of the temperature-responsive polymer include polymers that change from hydrophilic to hydrophobic upon a temperature change. Temperature-responsive polymers that can be used include known polymers and those similar thereto. Commercially available culture dishes precoated with a temperature-responsive polymer may be used. Cell sheets for preventing adhesion substantially consisting of mesothelial cell sheets containing no support can be obtained by using a culture dish coated with a temperature-responsive polymer.

The "mesothelial cells produced by a method for producing mesothelial cells according to the present invention" may be any of cells after the first culturing step, cells after the first sorting step, cells after the second culturing step, or cells after the second sorting step.

In one embodiment, a cell sheet for preventing adhesion according to the present invention contains 50% or more, 60% or more, 70% or more, 80% or more, 90% or more, 95% or more, 98% or more, or 99% or more of PCLP1-positive and ALCAM-positive cells.

A cell sheet for preventing adhesion containing liver mesothelial cells can be used for promoting the prevention of adhesion and/or regeneration of any organ or tissue and it is useful, for example, for prevention of adhesion of the resected surface after partial hepatectomy. Adipose tissue in the abdominal cavity attaches to the resected surface after partial hepatectomy and the surface is susceptible to adhesion. Currently, actively attempting "repeated hepatectomy" is the only treatment having a hope of radical cure or elongation of the survival term for relapse in the remnant liver after hepatectomy against primary liver cancer or liver metastasis of colon cancer. Adhesion produces the necessity of adhesiolysis upon the reoperation and the reoperation after the reoperation and increases the risk of massive hemorrhage and the infection. Moreover, adhesion also reduces hepatic functional reserve and thereby limits the number of the repeated hepatectomy and makes postoperative liver failure more likely to occur.

Use of cell sheets for preventing adhesion containing liver mesothelial cells can significantly prevent adhesion at the resected surface after partial hepatectomy, and also promote the liver regeneration to prevent the decrease of hepatic functional reserve.

The term "resected surface," as used herein, refers to a surface that appears by resection or ablation of a part of an organ or tissue.

The term "hepatic functional reserve," as used herein, means the function of the liver itself. The hepatic functional reserve can be evaluated by a known method. For example, liver damage classification and Child-Pugh classification are used often. The liver damage classification is a method of evaluation involving measuring ascites, serum bilirubin level, serum albumin level, ICG R15 and prothrombin activity and the Child-Pugh is a method of evaluation involving measuring encephalopathy, ascites, serum bilirubin, serum albumin, and prothrombin activity.

The phrase "promote liver regeneration, " as used herein, means achieving at least one of: improving at least one of the numerical values of ascites, serum bilirubin level, serum albumin level, ICG R15, prothrombin activity, and encephalopathy; improving the evaluation by the liver damage classification or the Child-Pugh classification; growing the liver; increasing the weight of the liver; promoting the proliferation of hepatocytes; and increasing the size of hepatocytes.

The cell sheet for preventing adhesion according to the present invention, especially when containing the extracellular matrix, does not require suturing, but takes just by placing or applying it on the surface of an organ or tissue or the resected surface.

Moreover, the cell sheet for preventing adhesion according to the present invention can be used for preventing adhesion of any organ or tissue in which adhesion may develop. For example, cell sheets for preventing adhesion made from liver mesothelial cells can be used for preventing adhesion in the intestine or the peritoneum and also cell sheets for preventing adhesion made from peritoneum mesothelial cells can be used for preventing adhesion of the liver resected surface.

In one embodiment, a method for preventing adhesion according to the present invention includes a step of placing the aforementioned cell sheet for preventing adhesion during a surgical operation on a surface or a resected surface of tissue or an organ in which adhesion may develop. The cell sheet for preventing adhesion can take on tissue or an organ and prevent adhesion just by placing it without requiring suturing or the like, but it may be sutured.

In one embodiment, a method for promoting liver regeneration according to the present invention includes a step of placing a cell sheet for preventing adhesion on a resected surface after partial hepatectomy. According to this method, postoperative liver failure can be prevented as well as the repetition number of hepatectomy can be increased, as a result of preventing adhesion, which is a problem in repeated hepatectomy, and promoting the recovery of hepatic functional reserve.

### Examples

### 1. Induction of differentiation from human iPS cells into liver mesothelial precursor cells (embryonic liver mesothelial cells)

Differentiation from human iPS cells into functional liver mesothelial precursor cells was induced.

Human iPS cells (obtained from RIKEN BRC) were cultured feeder-free on matrigel in the following medium to maintain an undifferentiated state.
Human iPS cell maintaining medium
StemSure (registered trademark) hPSC medium Δ (wako) 5 ng/mL bFGF (gibco)

Subsequently, undifferentiated human iPS cells were cultured in suspension in the following Mesoderm differentiation inducing medium supplemented with 5 ng/mL of BMP4. 1 to 3 days later, 5 ng/mL each of BMP4, Activin A and bFGF were further added and cultured for 1 to 5 more days at 37°C and 5% carbon dioxide to differentiate the cells into mesoderm.

### Mesoderm differentiation inducing medium

Iscove's Modified Dulbecco's Media (Life Technologies) 20% Fetal Bovine Serum
1% nonessential amino acids (Life Technologies)
1% L-glutamine penicillin streptomycin (Life Technologies)

To induce differentiation of mesodermal cells derived from human iPS cells into liver mesothelial precursor cells, the mesodermal cells derived from human iPS cells were then plated on culture dishes coated with collagen IV. For the first 2 to 7 days, the cells were cultured at 37°C and 5% carbon dioxide in the following Mesothelial precursor cell differentiation inducing medium.
Mesothelial precursor cell differentiation inducing medium
StemPro (registered trademark) -34 SFM medium (Life Technologies)
Insulin, Transferrin, Selenium, Ethanolamine Solution (Gibco)

Subsequently, 10 ng/mL each of OSM and bFGF were added to the Mesothelial precursor cell differentiation inducing medium and the cells were cultured for 1 to 3 weeks.

Using PCLP1 and ALCAM as markers, the percentage of ALCAM-positive and PCLP1-positive expression in the cells derived from the human iPS cells and induced for the differentiation was examined (Figure 2). The left panels in the Figure 2 illustrate the experimental result using isotype controls and the upper panels illustrate the results of single staining for PCLP1 and ALCAM, respectively. As indicated in the lower right panel, the PCLP1-positive and ALCAM-positive cells were 14.21% of the total cells.

Subsequently, the ALCAM-positive and PCLP1-positive fraction was sorted and purified with a cell sorter. The sorted cell population was further cultured (in the MEM alpha + GlutaMAX medium (invitrogen), 10% Fetal Bovine Serum, 50 nM 2-Mercaptoethanol (invitrogen), 1% L-glutamine penicillin streptomycin, 10 ng/mL bFGF, 10 ng/mL OSM). After the culturing, the morphology characteristic of liver mesothelial precursor cells (Figure 1, left panel), which is different from the morphology of undifferentiated human iPS cells (Figure 1, right panel) was observed.

### 2. Gene expression analysis of mesothelial precursor cells derived from human iPS cells

Expression of a variety of genes was analyzed in the liver mesothelial precursor cells produced by inducing differentiation from human iPS cells. The ratios to the expression level of each gene in the undifferentiated human iPS cells, which is defined to be 1, are indicated. From previous studies, liver mesothelial precursor cells are known to be characterized by their adhesion-preventing and liver regeneration-promoting effects. It was found that HGF, which is involved in the adhesion preventing effect, and HGF (hepatocyte growth factor), Ptn (pleiotrophin), Mdk (Midkine), and HB-EGF (heparin-binding EGF-like growth factor), which contribute to the liver regeneration, were expressed in the liver mesothelial precursor cells derived from human iPS cells (Figure 3).

### 3. Induction of differentiation from murine iPS cells into liver mesothelial precursor cells

### 3-1. Material and method

### <Murine iPS cells>

The GT3.2 murine iPS cells (miPS) were obtained from Dr. Kobayashi and Dr. Nakauchi at the University of Tokyo. The GT3.2 miPS cells were generated by introducing 3 factors into tail tip fibroblasts of an adult EGFP-transgenic C57BL/6 mouse with retroviral vectors (Cell, 2010, 142, 787-799).

### <Cell culture>

Undifferentiated GT3.2 miPS cells were cultured in the maintaining medium supplemented with LIF (Leukemia Inhibitory Factor), CHIR99021, and PD0325901 without using feeder cells on gelatine-coated culture dishes.

All cell cultures were conducted at 37°C and 5% CO₂.

### <Media>

### <iPS cell maintaining medium>

Glasgow's modified Eagle's medium (invitrogen)
10% Fetal Bovine Serum
0.1 mM 2-Mercaptoethanol (invitrogen)
0.1 mM nonessential amino acids (invitrogen)
1 mM sodium pyruvate (invitrogen)
1% L-glutamine penicillin streptomycin (sigma)
1000 U/ml mouse LIF (Millipore)
1 µM PD0325901 (Wako)
3 µM CHIR99021 (Millipore)

### <EB differentiation medium>

Glasgow's modified Eagle's medium (invitrogen)
10% Fetal Bovine Serum
0.1 mM 2-Mercaptoethanol (invitrogen)
0.1 mM nonessential amino acids (invitrogen)
1 mM sodium pyruvate (invitrogen)
1% L-glutamine penicillin streptomycin (sigma)

### <Mesoderm differentiation medium>

MEM alpha + GlutaMAX medium (invitrogen)
10% Fetal Bovine Serum
50 nM 2-Mercaptoethanol (invitrogen)
1% L-glutamine penicillin streptomycin (sigma)

### <Antibodies>

anti-mouse biotin PCLP1: MBL
anti-mouse ALCAM: eBioscience
anti-rabbit Ki67: Leica
anti-mouse Msln: made in the inventors' labolatory

### <In vitro differentiation from miPS cells to mesothelial cells>

To form embryoid bodies (EB), the maintaining medium was changed to EB differentiation medium. After culturing for 4 days, the cells were cultured in Mesoderm differentiation medium on culture dishes coated with type 4 collagen for 7 days.

The medium was changed to the MEM alpha + GlutaMAX medium (a differentiation medium) containing 10% fetal bovine serum (FBS), 50 nM mercaptoethanol, penicillin, streptomycin, glutamine, 10 ng/mL basic fibroblast growth factor (bFGF), and 10 ng/mL oncostatin M and the cells were cultured for 5 days.

To isolate cells derived from GT3.2 miPS cells and directed to liver mesothelium, GFP, mesothelin (Msln), and PCLP1-positive cells were sorted using a cell sorter. After the sorting, the cells were further cultured in the differentiation medium supplemented with Insulin-Transferrin-Selenium-X Supplement (ITS-X). To isolate liver mesothelial precursor cells derived from GT3.2 miPS cells, cells positive for all of GFP, PCLP1, and ALCAM were finally sorted with a cell sorter.

### <Flow cytometry>

To analyze the PCLP1-positive and ALCAM-positive cells, the cells differentiated from GF3.2 miPS cells were stained with a PE-conjugated ALCAM antibody and a biotin-conjugated PCLP1 antibody.

### <Functional analysis>

The cells differentiated from GT3.2 miPS cells were plated on Upcell plates (CellSeed Inc.) and cultured at 37°C. The plates were placed at room temperature to allow cells to detach from the plates and form cell sheets. The obtained cell sheets were grafted to resected surfaces in the PPHx operation of mice. The GFP signal was detected with a 130S-GFP camera (Science Eye). On Postoperative Day 10, the adhesion ratio was calculated according to the formula: maximum diameter of area in which adhesion developed/maximum diameter of resected surface. The size of hepatocytes and the number of Ki67-positive stem cells were measured with IN Cell Analyzer 2000 (GE Healthcare).

### 3-2. Results

### <Induction of differentiation from murine iPS cells into functional liver mesothelial precursor cells>

Differentiation from undifferentiated murine iPS cells into functional liver mesothelial precursor cells was induced.

First, undifferentiated murine iPS cells were cultured in suspension in EB differentiation medium to form EBs. The EBs were cultured under adherent conditions in Mesoderm differentiation medium on culture dishes coated with collagen type IV to differentiate into the mesoderm. Subsequently, cytokines (bFGF and oncostatin M) were added and the cells were cultured to differentiate into liver mesothelial cells. Only PCLP1-positive and Msln-positive cells derived from murine iPS cells and directed to the liver mesothelium were sorted with a cell sorter. The sorted cell population directed to the liver mesothelium was further cultured in the medium supplemented with ITS-X. The percentage of liver mesothelial precursor cells derived from murine iPS cells was analyzed with FACS by measuring the expression of embryonic liver mesothelial cell surface marker (ALCAM, PCLP1) as indicators. About 50% of cells were found to have differentiated into liver mesothelial precursor cells (Figure 5). ALCAM-positive and PCLP1-positive cells were sorted with a cell sorter and cultured. After the culturing, the morphology of monolayer squamous epithelium characteristic of embryonic liver mesothelial cells (Figure 4, left panel), which is different from the morphology of undifferentiated murine iPS cells (Figure 4, right panel), was observed uniformly.

### <Expression analysis of liver mesothelial precursor cells derived from murine iPS cells>

Expression of a variety of genes was then analyzed in the liver mesothelial precursor cells produced by inducing the differentiation from murine iPS cells. The ratios to the expression level of each gene in cultured embryonic liver mesothelium cells separated from the liver on Day 12.5 of pregnancy, which is defined to be 1, are indicated. The cytokines and growth factors considered to be characteristic of embryonic liver mesothelial cells from previous studies were mainly examined. It was found that PCLP1 and HGF, which are considered to be involved in the adhesion preventing effect, and HGF, EGF, Mdk, HB-EGF, and IL-6, which contribute to the liver regeneration, were expressed also in the liver mesothelial precursor cells derived from murine iPS cells (Figure 6).

### <Functional analysis of liver mesothelial precursor cells derived from murine iPS cells>

The present inventors have already reported that embryonic liver mesothelial cells separated from mice on Day 12.5 of pregnancy and cultured have both the posthepatectomy adhesion-preventing effect and the liver regeneration-promoting effect. It was examined whether the liver mesothelial precursor cells induced to differentiate from murine iPS cells have the two aforementioned functions specific for embryonic liver mesothelial cells.

The liver mesothelial precursor cells derived from murine iPS cells were cultured on temperature-responsive culture dishes and sheets of liver mesothelial precursor cells derived from murine iPS cells were produced.

A mouse model of hepatectomy adhesion (PPHx) was generated by the following procedure. First, under a general anesthetic, wild type C57BL/6J mice were each placed in a spin position. The mouse was incised along a median line 2 cm above from Xyphoid inferiorly. The peritoneal was incised with lifting up the peritoneal so as not to damage the intraperitoneal organs. After laparotomy, the falciform ligament was separated to the superior vena cava. The pedicle of the median lobe was tied with 4-0 silk and cut off to remove the median lobe. The hepatic capsule around the left lobe was then cauterized with an electric scalpel and the left lobe was resected at the middle thereof. During excision, the left portal veins and the left hepatic veins were ligated en bloc and separated.

Subsequently, a sheet of liver mesothelial precursor cells derived from murine iPS cells were applied to the liver resected surface of the mouse model of posthepatectomy adhesion and the adhesion preventing effect was first examined. On Postoperative Day 10, significant suppression of adhesion was observed in comparison with the non-application group. In addition, this adhesion preventing effect was as good as that of embryonic liver mesothelial cells derived from mice on Day 12.5 of pregnancy (Figure 7). It was demonstrated that adhesion can be alleviated also on Postoperative Day 30, as well as on Postoperative Day 10, by application of a sheet of liver mesothelial precursor cells derived from murine iPS cells (Figure 8).

For the liver regeneration promoting effect, the extent of liver regeneration was then examined in terms of the division of hepatocytes. The number of Ki67-positive hepatocytes was increased in the application group of sheet of liver mesothelial precursor cells derived from murine iPS cells in comparison with that in the non-application group and the quantification by imaging cytometry revealed that proliferation of hepatocytes was promoted in the application group (Figure 9).

Based upon the foregoing, it is considered that differentiation from murine iPS cells to functional liver mesothelial precursor cells was successfully induced.

## Claims

1. A method for producing mesothelial cells, comprising a first culturing step of culturing mesodermal cells in a medium containing basic fibroblast growth factor (bFGF) and oncostatin M.

2. The method for producing mesothelial cells according to claim 1, further comprising, after the first culturing step, a first sorting step selected from the following (i) and (ii) :
(i) a step of sorting podocalyxin-like protein 1 (PCLP1)-positive and mesothelin-positive cells; and
(ii) a step of sorting PCLP1-positive and activated-leukocyte cell adhesion molecule (ALCAM)-positive cells.

3. The method for producing mesothelial cells according to claim 2, further comprising, after the first sorting step, a second culturing step of culturing the cells in a medium containing bFGF and oncostatin M.

4. The method for producing mesothelial cells according to claim 3, wherein the medium of the second culturing step further comprises insulin-transferrin-selenium-X (ITS-X).

5. The method for producing mesothelial cells according to claim 3 or 4, wherein the first sorting step is the step of (i) and the method further comprises, after the second culturing step, a second sorting step of sorting PCLP1-positive and ALCAM-positive cells.

6. The method for producing mesothelial cells according to any one of claims 1 to 5, wherein the mesothelial cells are liver mesothelial cells.

7. The method for producing mesothelial cells according to any one of claims 1 to 6, wherein the mesodermal cells are cells differentiated in vitro from pluripotent stem cells.

8. The method for producing mesothelial cells according to claim 7, wherein the in vitro differentiation from the pluripotent stem cells to the mesodermal cells is achieved by suspension culture.

9. The method for producing mesothelial cells according to claim 7 or 8, wherein the in vitro differentiation from the pluripotent stem cells to the mesodermal cells is achieved such that embryoid bodies are formed.

10. A cell population of mesothelial cells produced by a method according to any one of claims 1 to 9, wherein the cell population comprises 10% or more of PCLP1-positive and ALCAM-positive cells.

11. A method for producing a cell sheet for preventing adhesion, comprising the steps of culturing the cell population according to claim 10 in a medium containing bFGF and oncostatin M; and producing a cell sheet with the cell population.

12. The method for producing a cell sheet for preventing adhesion according to claim 11, wherein the medium further comprises ITS-X.

13. A method for producing a cell sheet for preventing adhesion, comprising the steps of: selecting PCLP1-positive cells from cultured cells; and producing a cell sheet from the selected cells.

14. A method for producing a cell sheet for preventing adhesion, comprising the steps of: selecting PCLP1-positive and ALCAM-positive cells from cultured cells; and producing a cell sheet from the selected cells.

15. A cell sheet for preventing adhesion produced by a method according to any one of claims 11 to 14.

16. A cell sheet for preventing adhesion, consisting of a cell sheet comprising 50% or more of PCLP1-positive and ALCAM-positive cells.
